# EUROPEAN PATENT APPLICATION

(11) **EP 2 273 265 A1**
(43) Date of publication of application: **12.01.2011**
(21) Application number: 09164613.3
(22) Date of filing: 06.07.2009
(51) Int. Cl.: G01N 33/497

(54) **Method for the diagnosis of asthma by detecting volatile organic compounds in exhaled air.**

(71) Applicant: Universiteit Maastricht, 6211 LK Maastricht (NL); Academisch Ziekenhuis Maastricht, 6229 HX Maastricht (NL)
(72) Inventor: Schooten, Van, Frederik Jan, 6247 BB, GRONSVELD (NL); Dallinga, Jan Willem, 6411 VA, HEERLEN (NL); Berkel, Van, Joep Joseph Benjamin Nathan, 6224 CS, MAASTRICHT (NL)
(74) Representative: Habets, Winand

(57) **Abstract**

The present invention relates to the identification of markers for the disease conditions related to asthma. The uses of such markers in diagnosis and a novel method for their identification are herein described. We were able to discriminate between patients with asthma and normal individuals by determining whether the exhaled air contained markers selected from that set. We also found that these markers can be used in the early diagnosis of asthma. Moreover, these markers are useful in the prediction of the occurrence of asthma even when no other clinical signs are apparent yet.

## Description

### Field of the invention

The present invention relates to the identification of markers for disease conditions related to asthma. The uses of such markers in diagnosis and a novel method for their identification are disclosed herein.

### Background of the invention

Asthma is a frequently occurring chronic inflammatory disease in children world-wide. Although effective therapies are available, asthma control is still not optimal in many patients [Rabe KF, et al., J Allergy Clin Immunol 2004; 114:40-47, Wolfenden LL, et al., Arch Intern Med 2003;163: 231-236].

Until now, the criteria for the diagnosis and management of asthma are based on clinical observations and lung function tests [Global Initiative for asthma (GINA). Pocket guide for asthma management and prevention in children. National Institute of Health, National Heart, Lung, and Blood Institute 2002, Kharitonov SA, and Barnes PJ. Am J Respir Crit Care Med 2001;163:1693-1722]. However, symptoms may not always accurately represent the nature and extent of the underlying airway condition [Kharitonov SA. Swiss Med Wkly 2004;134:175-192] and lung function tests are not definite in relation to airway inflammation [Silvestri M, et al., Pediatr Pulmonol 2003;35:358-363 Sutherland ER, et al., J Allergy Clin Immunol 2004;113:1046-1050, Leung TF, et al., Thorax 2005;60:822-826, Rosias PP, et al., Pediatr Pulmonol 2004; 38:107-114].

Young children showing symptoms like coughing, wheezing and dyspnoea may be suffering from various diseases of the respiratory tract, and it is therefore not always easy to reach the proper diagnosis [Rosias PPR et al., Pediatr Allergy Immunol 2004;15:4-19, Chung KF. Eur Respir Rev 1998;8:999-1006, Helms PJ. Pediatr Pulmonol 2001;S21:49-56, Hunt J. J Allergy Clin Immunol 2002;110:28-34, Kharitonov SA, and Barnes PJ. Biomarkers 2002;7:1-32]. New diagnostic approaches with the potential to make an early diagnosis of asthma possible, may not only greatly decrease under-diagnosis and under-treatment in true asthmatics, but also prevent over-treatment in transient wheezers.

In recent years non-invasive techniques that may be useful for the assessment of airway inflammation have been found in the analysis of exhaled breath. For instance, the collection and analysis of non-volatile compounds in exhaled breath condensate [Rosias PPR et al., Pediatr Allergy Immunol 2004;15:4-19] and of volatile compounds such as nitric oxide (NO) [Ashutosh K. Curr Opin Pulm Med 2000;6:21-5, Baraldi E, et al., Pediatr Pulmonol 2004; Suppl.26:16-19, Cicutto LC, and Downey GP. ACN Clin Issues 2004;15:97-111, Malmberg LP, et al., Thorax 2003;58:494-499] and carbon monoxide (CO) [Paredi P, et al., Thorax 1999;54:917-920, Antuni JD, et al., Thorax 2000;55:138-142, Paredi P, et al., Am J Respir Crit Care Med 2002;166:S31-S37] in breath have been used to assess respiratory diseases. Fractional exhaled nitric oxide (FeNO) can be considered as a non-invasive marker of inflammation because its levels are elevated in children with asthma [Kharitonov SA, and Barnes PJ. Eur Respir J 2000;16:781-792, Nelson BV, et al., J Pediatr 1997;130:423-427, Frank TL, et al., Am J Respir Crit Care Med 1998; 158:1032-1036]. However, FeNO mainly represents eosinophilic inflammation, which may be present in different clinical conditions and therefore its discriminating power is limited [Prasad A, et al., Respir Med 2006;100:167-173, Gratziou C, et al., Eur Respir J 1999;14:897-901].

Also, ethane and pentane which are detectable volatile organic compounds (VOC) in exhaled air have been related to oxidative stress as a result of inflammatory conditions [Paredi P, et al., Am J Respir Crit Care Med 2002;166:S31-S37, Olopade CO, et al., Chest 1997;111:862-865, Paredi P, et al., Am J Respir Crit Care Med 2000;161:1247-1251]. In all these approaches, single or small numbers of components are analysed and attempted to be used as diagnostic tools, so far with limited success because such individual markers suffered from a low sensitivity and/or specificity.

### Summary of the invention

We analyzed the volatile organic compounds (VOCs) in exhaled air of patients with asthma by using a gas chromatograph - time of flight - mass spectrometer (GC-TOF-MS). We found a set of markers that may be used for the diagnosis of asthma.

We were able to discriminate between patients with asthma and normal individuals by determining whether the exhaled air contained markers selected from that set.

More in particular we found that these markers can be used in the early diagnosis of asthma. Moreover, these markers are useful in the prediction of the occurrence of asthma even when no other clinical signs are apparent yet.

The method is also useful since it provides a new non-invasive, easy, safe, cost effective and fast diagnostic tool for the diagnosis of asthma. Furthermore, the method may be useful to distinguish between healthy wheezers and asthmatic children.

Hence, the invention relates to an in vitro method of diagnosing asthma in a patient by determining the amount of at least one volatile organic compound in a sample derived from a patient suspected of having asthma and comparing said amount with a normal value for said volatile organic compound, wherein a difference between the normal value and the amount of said at least one volatile organic compound is indicative of asthma, wherein said volatile organic compound is selected from the group consisting of hydrocarbon C₁₃H₂₈, carbon disulfide (CS₂), 1-penten-2-on, butanoic acid, 3-(1-methylethyl)-benzene, hydrocarbon C₁₃H₂₈, unsaturated hydrocarbon C₁₅H₂₆, benzoic acid, p-xylene, hydrocarbon C₁₁H₂₄.

### Detailed description of the invention

It was possible to assess distinctive VOC profiles, which discriminated between asthma patients and controls. Based on 20 times cross validation, a correct classification of asthma patients and controls was found using a VOC selected from table 1.

**TABLE 1**

| *Identification of 10 most discriminating attributes between asthma and controls* | | | |
|---|---|---|---|
| **VOC No:** | **Identified as** | **Chemical formula** | **Retention Time [min.]** |
| 1 | (branched) hydrocarbon | C₁₃H₂₈ | 18.89 |
| 2 | carbon disulfide | CS₂ | 2.99 |
| 3 | 1-penten-2-on | C₅H₈O | 6.29 |
| 4 | butanoic acid | C₄H₈O₂ | 9.26 |
| 5 | 3-(1-methylethyl)-benzene | C₉H₁₂ | 14.78 |
| 6 | (branched) hydrocarbon | C₁₃H₂₈ | 19.00 |
| 7 | unsaturated hydrocarbon | C₁₅H₂₆ | 22.78 |
| 8 | benzoic acid | C₇H₆O₂ | 16.87 |
| 9 | p-xylene | C₈H₁₀ | 11.66 |
| 10 | (branched) hydrocarbon | C₁₁H₂₄ | 16.93 |

The compounds in table 1 can be distinguished on the basis of their chemical formula only, or in a number of cases by the combination of their chemical formula and the retention time of the compound on the specific column used (see examples section). Under the circumstances used, the retention time of toluene was 9.15 minutes. The skilled person will appreciate that the retention times as referred herein are approximate values subject to the usual experimental variance.

Hence, the invention relates to an in vitro method of diagnosing asthma in a patient by determining the amount of at least one volatile organic compound in a sample derived from a patient suspected of having asthma and comparing said amount with a normal value for said volatile organic compound, wherein a difference between the normal value and the amount of said at least one volatile organic compound is indicative of asthma, wherein said volatile organic compound is selected from the group consisting of hydrocarbon C₁₃H₂₈, carbon disulfide (CS₂), 1-penten-2-on, butanoic acid, 3-(1-methylethyl)-benzene, hydrocarbon C₁₃H₂₈, unsaturated hydrocarbon C₁₅H₂₆, benzoic acid, p-xylene, hydrocarbon C₁₁H₂₄.

Said sample derived from a patient is preferably a sample of exhaled air from the patient, it may also be a sample wherein the volatile organic compounds in the exhaled air are concentrated, collected and/or immobilized, such as an active carbon solid phase or an affinity matrix.

A method according to the invention makes a contribution to the art as a whole, in that it provides a method with a sensitivity of more than 45% while the specificity is more than 85%. The prior art methods for diagnosing asthma that measure volatile organic compounds in exhaled air do not provide such high specificity and/or sensitivity.

Methods to detect VOCs in exhaled air as such are known in the art. The examples provide guidance for the skilled person to perform an analysis according to the invention. Normal values may be obtained by determining VOCs in the exhaled air of normal individuals. VOCs may be detected in several ways known in the art. This may be done by a device capable of measuring more than one VOC at the same time such as a mass spectrometer or by a device specifically suitable for measuring only one VOC at a time.

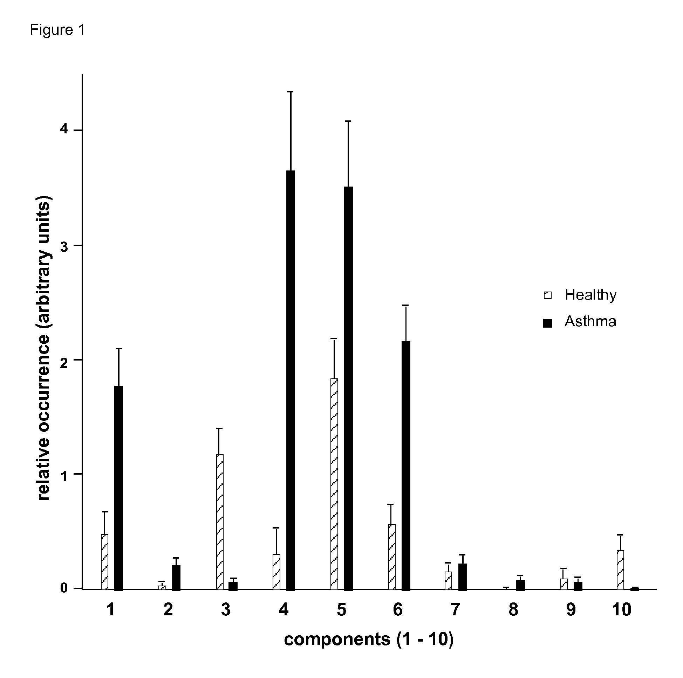

Figure 1 shows the mean relative intensity of the ten VOCs from table 1. It can be seen that VOCs 1, 2, 4, 5, 6, 7, and 8 increased in the population of asthma patients, whereas VOC numbers 3, 9 and 10 are decreased in the population of asthma patients when compared to normal controls.

When a single VOC is to be used, VOC No 1, i.e. a hydrocarbon with molecular formula of C₁₃H₂₈ and retention time of 18.89 min is preferred. This may be advantageous in an assay capable of detecting only a single VOC at a time such as an electronic nose. The preferred hydrocarbon may be linear or branched.

The sensitivity, specificity, and the percentage of correct classification increased with increasing number of VOCs selected from table 1. Hence, the invention also relates to a method as described above wherein at least two volatile organic compounds are detected, such as three, four, five or six VOCs. The method may even be further improved by determining the relative amounts of seven, eight, nine VOCs. Most preferred is a method as described above wherein all ten VOCs from table 1 are determined in a method according to the invention.

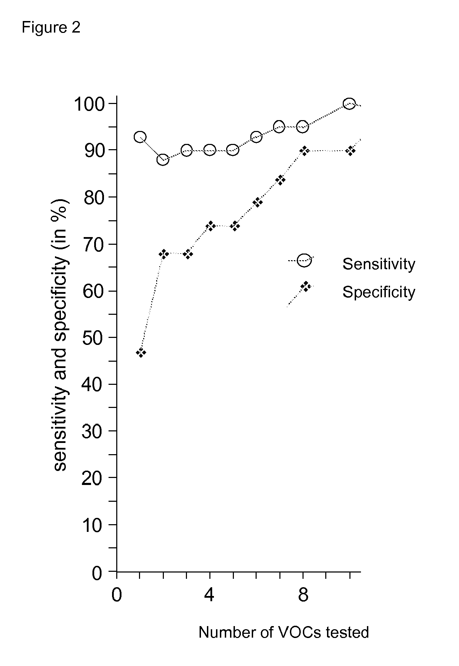

Figure 2 shows the percentage of correct classification of asthma with increased numbers of VOCs. It can be seen that the percentage of correct classification increases when more than one VOC selected from table 1 is used in a method according to the invention. The highest sensitivity and specificity could be obtained when a method according to the invention was performed with all 10 VOCs from table 1 combined.

A preferred embodiment of the method according to the invention comprises the use of VOC No 1 together with a VOC selected from the group consisting VOC No 2 to VOC No. 10, preferably No 2.

When the discriminant analyses was performed on 95% of the chromatograms (training set), leaving the remaining 5% (test set) as unknowns to be classified using the training model, 6 VOCs were sufficient to reach a sensitivity and a specificity of both 84%, 8 VOCs were sufficient to reach 89% sensitivity at 95% specificity whereas 100% sensitivity could be reached when all VOCs from table 1 were used (figure 2).

VOC can easily be determined by collection of exhaled breath and quantitative analysis by thermal desorption-gas chromatography time-of-flight mass spectrometry (GC-TOF-MS) [Van Berkel JJBN, et al., J Chrom B 2008; 861:101-7].

A method according to the invention may be particularly advantageous for the diagnosis of asthma in children below 6 years of age, being an age group in which a definitive diagnosis based on doctors' examination is still difficult. Since under-diagnosis and under-treatment in true asthmatics and over-treatment in transient wheezers are undesirable from a medical standpoint and in terms of public health, an earlier diagnosis would be valuable.

A method according to the invention may also be used to distinguish atopic asthma patients from healthy persons The results of the analyses in which breath samples of atopic and non-atopic asthma patients were compared, indicate that not only healthy from diseased can be distinguished in this way, but that it is also possible to differentiate one form of disease from another.

### Legend to the figures

Figure 1 shows the mean relative intensities of the ten VOCs listed in table 1which were capable to distinguish between asthmatic and healthy individuals.
Figure 2 shows the percentage of correct classification of asthma with increasing numbers of VOCs.

### Examples

### Example 1: Study population

A total of 120 children in the age range of 5 - 16 years were included in the study. Of these children 63 were diagnosed with asthma and 57 were healthy controls. The asthmatic children were recruited from the outpatient clinic of the department of paediatric pulmonology of the University Hospital Maastricht. The asthma diagnosis was made on clinical grounds by an experienced children's lung specialist.

Asthma was defined as a chronic inflammatory disorder with intermittent symptoms of cough, dyspnoea, wheezing and chest pain. Severity and control of asthma were assessed according to the international Global Initiative for Asthma (GINA) guidelines [Global Initiative for asthma (GINA). Pocket guide for asthma management and prevention in children. National Institute of Health, National Heart, Lung, and Blood Institute 2002].

A subset of 42 atopic asthma patients was selected, based on an established positive Phadiatop and/or at least two RAST classes equal to or greater than 2. Appropriate therapy was prescribed by the patient's own physician. All 63 patients used an inhaled bronchodilator on demand and 55 of them used an inhaled corticosteroid daily. Sixty seven percent of this population had allergy as documented by radio allergosorbent test (RAST) class 2 or higher for at least one common airborne allergen.

The 57 control subjects were recruited from primary schools (36 out of 57) and from the outpatient paediatric clinic of the University Hospital Maastricht (21 out of 57). In this group enuresis and constipation were the main reasons for consultation.

The ISAAC questionnaire was used to exclude a history of respiratory problems in these children [Variations in the prevalence of respiratory symptoms, self-reported asthma attacks and use of asthma medication in the European Community Respiratory Health Survey (ECRHS). Eur Respir J 1996; 9:687-95]. Patients with diseases that might interfere with the results of this study, mentally retarded children and active smokers were excluded from the study. The two populations were comparable in age, height and weight.

Characteristics of patients and controls are presented in table 2. Informed consent was obtained from all children and their legal representatives. The study was approved by the Ethics Committee of Maastricht University, clinical trial registration number NCT 00413140.

**Table 2 Characteristics of the study population**

| | Asthma (n=63) | Controls (n=57) |
|---|---|---|
| Age (mean ± SD) | 10.8 ± 3.1 | 9.9 ± 2.8 |
| Height | 144 ± 17 | 141 ± 17 |
| Weight | 38.2 ± 14.9 | 34.8 ± 10.9 |
| Gender male/female | 47 / 16 | 29 / 28 ⁽²⁾ |
| FEV₁ (% predicted) | 96.2 ± 18.2 | 101.8 ± 10.6 |
| FEV₁ / VC (%) | 84.0 ± 10.3 | 88.2 ± 7.0 |
| Atopy | 42 (67%) | |
| Asthma severity ⁽¹⁾ | | |
| Intermittent | 15 (24%) | |
| mild persistent | 12 (19%) | |
| moderate persistent | 17 (27%) | |
| severe persistent | 19 (30%) | |
| allergic rhinitis | 11 (17%) | |
| ICS use | 55 (87%) | |
| ICS daily dose (µg) | 576 ± 357 | |

| | | |
|---|---|---|
| ⁽¹⁾Severity was classified according to Global Initiative for Asthma (GINA) ⁽²⁾ Significantly different (p=0.01). No confounding of gender on the discriminant functions for asthma vs. control and atopic vs. non-atopic asthma could be established. | | |

### Example 2: Sampling

The subjects were asked to exhale their breath in a resistance free plastic bag (Tedlar bag, SKC Itd., Dorset, UK). Three to four exhalations were sufficient to fill the 5-litre bag. Neither special provisions for the manner of exhalation, nor special directions concerning the diet of the subjects were given. The samples of both, patients and controls were collected in the same room.

Within one hour of collection, the bag was emptied under pressure over a stainless steel two-bed sorption tube, filled with carbograph 1TD/Carbopack X (Markes International, Llantrisant, Wales, UK). Before and after loading the tubes were air-tight capped. The tubes were stored at room temperature until analysis.

All patients were instructed to withhold from using their inhaler during 8 hours prior to the sampling.

### Example 3: VOC Analysis

Analysis of the samples was performed as previously described [Van Berkel JJBN, et al., J Chrom B 2008;861:101-7]. In brief; the volatile compounds trapped on the sorption tubes by thermal desorption were released, using the Markes International Ultra-Unity automated thermal desorption equipment (Markes International, Llantrisant, Wales, UK). The gaseous mixture of released compounds was then split; 90% of the sample was recollected on a second identical sample tube and stored for an optional second analysis. 10% of the sample was loaded onto a cold (5 °C) sorption trap, from which it was injected into a gas chromatograph (Trace GC, Thermo Fischer Scientific, Austin, Texas, USA) and analysed by time-of-flight mass spectrometry (Tempus Plus, Thermo Fischer Scientific, Austin, Texas, USA).

For the GC-MS measurements the following conditions were used: column: Restek RTX-5ms, 30m x 0.25 mm ID, coated with 1.0 um HP-5 phase; helium was used as the carrier gas at a flow rate of 1.5 ml / min. The temperature of the gas chromatograph was programmed as follows: 40 °C for 5 min., then increased by 10 °C/min until 270 °C, at which temperature it was maintained for 5 min. The mass spectrometer was set at a scan range of 35-350 AMU and scanned 5 times per second. The complete analytical procedure, including sampling, storage and instrumental analysis was tested for reproducibility. Instrumental reproducibility was determined by the analysis of identical exhaled air samples that were obtained by emptying a filled bag over γ-shaped connector onto two absorption tubes. The two absorption tubes were subsequently analyzed by GC-TOF-MS. This experiment was repeated 6 times.

The quantification of the similarity was done by means of calculation of a distance measure (dot product rule). This distance measure is based on the similarity of the entire raw chromatogram. Distance measure calculation of all complementary files resulted in a distance measure ranging from 0.96 to 0.99. (A value of '1' denotes identical samples, the lower the value the lesser the degree of similarity). Intra-individual variability was examined by repeated sampling of exhaled air from 10 subjects for 5 consecutive days and comparing the results per subject from day to day. Inter-individual variability was examined by sampling 10 subjects and comparing the data from subject to subject. Again the similarity between several chromatograms was quantified using a distance measure as mentioned above. The intra-individual variability ranged from 0.80 to 0.99 and is far smaller than the inter-individual variability, ranging from 0.16 to 0.98

### Example 4: Data analysis

The GC-MS chromatograms of the breath samples of the 120 subjects involved in this study were recorded and retention times were normalized by calculating retention indices, relative to toluene and lining up using easily recognizable component peaks, to correct for chromatographic drifting. Parts of the chromatograms that occurred at a retention index < 0.15 and at a retention index > 2.8 were removed from the chromatograms, because of unreliable data from these parts, due to noisy mass spectra at the beginning of the chromatograms and column bleeding at the end of each run.

The remaining data were transformed to Excel files, containing almost 6000 different chromatographic peaks, determined by retention time and mass spectrum and combined with a relative intensity. In our approach the measured mass spectra were compared to one another at the same retention time, rather than to library spectra.

In this way the resemblance of the original spectra determined the decision whether peaks at the same retention time represent the same component or not. Intensities below the detection limit were set at 0%. The detailed descriptions of the data handling procedures can be found elsewhere [Van Berkel JJBN, et al., J Chrom B 2008;861:101-7.].

Before data analysis was executed, all peaks that occurred in less than 8% of the samples (meaning less than ten times in this study) were removed. This resulted in a final data matrix, containing 945 peaks and 120 subjects. This matrix was normalized by adjusting the sum of all peak intensities in each subject at 100%. This way the influence of the infrequently appearing peaks could be diminished. The resulting data were analysed by a stepwise discriminant analysis, using SPSS (SPSS13.0 for windows, SPSS Inc. Chicago, Illinois, USA).

The discriminant analyses were performed using a twentyfold crossover approach. In this method all but 5 percent of the chromatograms (in this study 5 percent equals 6 samples) are used to construct the discriminant function, which is subsequently used to predict to which group the ones left out belong. This is repeated 20 times, until all samples have once been classified.

## Claims

1. In vitro method of diagnosing asthma in a patient by determining the amount of at least one volatile organic compound in a sample derived from a patient suspected of having asthma and comparing said amount with a normal value for said volatile organic compound, wherein a difference between the normal value and the amount of said at least one volatile organic compound is indicative of asthma, wherein said volatile organic compound is selected from the group consisting of hydrocarbon C₁₃H₂₈, carbon disulfide (CS₂), 1-penten-2-on, butanoic acid, 3-(1-methylethyl)-benzene, hydrocarbon C₁₃H₂₈, unsaturated hydrocarbon C₁₅H₂₆, benzoic acid, p-xylene, hydrocarbon C₁₁H₂₄.

2. Method according to claim 1 wherein said method comprises the step of determining at least the amount of a hydrocarbon C₁₃H₂₈in said sample.

3. Method according to claim 2 wherein said method consists of the step of determining at least the amount of a hydrocarbon C₁₃H₂₈in said sample

4. Method according to claims 1 or 2 wherein at least 2 volatile organic compounds are detected.

5. Method according to claim 4 wherein at least 3 volatile organic compounds are detected.

6. Method according to claim 5 wherein at least 4 volatile organic compounds are detected.

7. Method according to claim 6 wherein at least 5 volatile organic compounds are detected.

8. Method according to claim 7 wherein at least 6 volatile organic compounds are detected.

9. Method according to claim 8 wherein at least 7 volatile organic compounds are detected.

10. Method according to claim 9 wherein at least 8 volatile organic compounds are detected.

11. Method according to claim 10 wherein at least 9 volatile organic compounds are detected.

12. Method according to claim 11 wherein at least 10 volatile organic compounds are detected.
